(19) Europäisches Patentamt
European Patent Office
Office européen des brevets

(11) **EP 3 791 776 B1**

(12) **FASCICULE DE BREVET EUROPEEN**

(45) Date de publication et mention de la délivrance du brevet:
**22.02.2023 Bulletin 2023/08**

(21) Numéro de dépôt: **19197212.4**

(22) Date de dépôt: **13.09.2019**

(51) Classification Internationale des Brevets (IPC):
**A61B 5/00** *(2006.01)*    **A61B 5/083** *(2006.01)*
**A61B 5/22** *(2006.01)*

(52) Classification Coopérative des Brevets (CPC):
**A61B 5/4866; A61B 5/0833; A61B 5/22;**
**A61B 5/222; A61B 5/4884**

(54) **PROCEDE DE DETERMINATION DES PARAMETRES METABOLIQUES D'UNE PERSONNE**

VERFAHREN ZU BESTIMMUNG DER STOFFWECHSELPARAMETER EINER PERSON

METHOD FOR DETERMINING THE METABOLIC PARAMETERS OF A PERSON

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**17.03.2021 Bulletin 2021/11**

(73) Titulaires:
• **Université Paris Cité**
  **75006 Paris (FR)**
• **Université Paris-Saclay**
  **91190 Gif-sur-Yvette (FR)**
• **Centre National de la Recherche Scientifique**
  **75016 Paris (FR)**

(72) Inventeurs:
• **GOUPIL, Christophe**
  **92110 Clichy (FR)**
• **LECOEUR, Philippe**
  **91440 Bures sur Yvette (FR)**
• **HERBERT, Eric**
  **75020 Paris (FR)**
• **BELS, Vincent**
  **22130 Pleven (FR)**
• **OUERDANE, Henri**
  **121205 Moscou (RU)**

(74) Mandataire: **A.P.I. Conseil**
**Immeuble Newton**
**4, rue Jules Ferry**
**64000 Pau (FR)**

(56) Documents cités:
• **DAIJIRO ABE ET AL: "Energy cost and lower leg muscle activities during erect bipedal locomotion under hyperoxia", JOURNAL OF PHYSIOLOGICAL ANTHROPOLOGY, BIOMED CENTRAL LTD, LONDON, UK, vol. 37, no. 1, 19 juin 2018 (2018-06-19), pages 1-11, XP021257585, DOI: 10.1186/S40101-018-0177-7**
• **H. J. RALSTON: "Energy-speed relation and optimal speed during level walking", EUROPEAN JOURNAL OF APPLIED PHYSIOLOGY, vol. 17, no. 4, 1 octobre 1958 (1958-10-01), pages 277-283, XP055647368, DE ISSN: 1439-6319, DOI: 10.1007/BF00698754**
• **CHRISTOPHE GOUPIL ET AL: "Thermodynamics of metabolic energy conversion under muscle load", NEW JOURNAL OF PHYSICS, vol. 21, no. 2, 1 février 2019 (2019-02-01), page 023021, XP055647363, GB ISSN: 1367-2630, DOI: 10.1088/1367-2630/ab0223**

**Description**

**DOMAINE DE L'INVENTION**

La présente invention concerne un procédé et un dispositif de caractérisation de la réponse métabolique d'un sujet à l'effort.

**ETAT DE LA TECHNIQUE**

L'étude de la réponse à l'effort est un sujet ancien qui couvre de nombreux domaines dont les principaux sont la physiologie à l'effort, et plus spécifiquement la physiologie du sport, ainsi que la biomécanique.

La dépense énergétique est connue et étudiée depuis la fin du 19ème siècle, y compris du point de vue thermodynamique via une approche calorimétrique, puis métabolique et nutritionnelle.

Les études sur la ventilation et l'adaptation ventilatoire sont elles aussi très largement répandues et sont intégrées dans les tests d'effort.

L'ouvrage des auteurs Monod, Flandrois et Vandewalle (Physiologie du sport 6ème édition Masson (2007)), fait une recension exhaustive de ces questions.

Les publications suivantes donnent des exemples de protocoles d'études correspondants:

- D. Abe et al: "Energy cost and lower leg muscle activities during erect bipedal locomotion under hyperoxia", Journal of Physiological Anthropology, BIOMED CENTRAL L TD, LONDON, UK, vol. 37, no. 1, 19 juin 2018 (2018-06-19), pages 1-11;
- H. J. Ralston: "Energy-speed relation and optimal speed during level walking",European Journal of Applied Physiology, vol. 17, no. 4, 1 octobre 1958 (1958-10-01), pages 277-283.

On notera que les principaux protocoles d'identification du potentiel énergétique d'un individu sont basés sur les mesures suivantes :

- Ventilation, de laquellle sont extraites les courbes de Cost of Oxygen Transport, (COT) ;
- Débit et fréquence cardiaque (ECG), gaz du sang ;
- Réponse force-vitesse de muscles (associée au modèle de Hill 1938 et 1964) ;
- Electromyogrammes.

Cette liste permet d'identifier que chacune de ces mesures nécessite un appareillage spécifique.

Par ailleurs, la réalisation d'une cartographie synthétique n'est accessible qu'au prix de protocoles multiples sur des équipements différents.

Par ailleurs, la publication (Christophe Goupil et al 2019 New J. Phys.21 023021 « Thermodynamics of metabolic energy conversion under muscle load ») pose les bases thermodynamiques de la réponse d'un muscle, ou d'un ensemble de muscles, soumis à effort.

Il en ressort que, moyennant une mesure classique de la réponse force-vitesse d'un muscle, deux paramètres métaboliques peuvent être estimés : la force isométrique $F_{iso}$, d'une part, et une résistance de contre-réaction, $R_{fb}$, d'autre part.

**EXPOSE DE L'INVENTION**

Un but de l'invention est de permettre de quantifier l'état de santé d'un individu à partir d'un protocole expérimental composé de deux épreuves. Le caractère innovant provient de la description thermodynamique de la réponse à l'effort, qui permet de rassembler les résultats de ces épreuves dans un cadre unique de description.

Le procédé et le dispositif permettent d'obtenir, au cours d'un protocole de test unique, une cartographie de la performance et des potentialités énergétiques d'un sujet par identification directe de sa réponse musculaire couplée à sa réponse ventilatoire.

A ce jour, il n'existe pas de protocole unique permettant d'obtenir cette caractérisation directement. Les mesures et les protocoles existants sont réalisés séparément et ne permettent pas d'obtenir une cartographie synthétique de la réponse à l'effort d'un individu ou d'un sujet.

A l'issue du protocole réalisé avec le dispositif, la cartographie synthétique se compose des 5 éléments suivants :

- Puissance basale, notée B
- Intensité métabolique de seuil, notée $I_T$

- Résistance métabolique, notée $R_M$
- Résistance de rétroaction, notée $R_{fb}$
- Force isométrique, notée $F_{iso}$

L'ensemble de ces paramètres permet d'obtenir la valeur du facteur de mérite métabolique, $F_m=(F_{iso}\,R_{fb}\,I_T)/(B\,R_M)$, qui est l'indicateur principal de la potentialité de l'individu.

A ce titre, le dispositif proposé peut à la fois servir au suivi de la performance sportive, ainsi qu'à la rééducation fonctionnelle.

Plus précisément, l'invention, telle que définie dans la revendication indépendante 1, présente un procédé de détermination de paramètres métaboliques d'une ou plusieurs unités motrices musculaires d'un sujet par couplage de sa réponse musculaire à sa réponse ventilatoire,

Comprenant, les étapes suivantes :

(i)+(ii)-une étape d'acquisition , par des moyens d'acquisition, en fonction du temps d'une vitesse de déplacement d'une ou plusieurs unités motrices musculaires d'un sujet v(t), et d'un débit d'oxygène $\varphi_{O2}$ (t) du sujet ;

(iii)-une étape de tracé, par des moyens de calcul, de la courbe COT entre le débit d'oxygène du sujet et la vitesse de déplacement v(t)d'une ou plusieurs unités motrices musculaires du sujet v(t),

la courbe COT étant couplée à la réponse musculaire par l'équation suivante :

$$COT = \frac{\Phi_-}{v} = a_0 + R_M v + \frac{B}{v}$$

Avec B la puissance basale d'une ou plusieurs unités motrices musculaires $R_M$ la résistance d'écoulement d'une ou plusieurs unités motrices musculaires

(iv)-une étape de détermination , par les moyens de calcul, des paramètres métaboliques suivants à partir de cette courbe COT :

- Puissance basale B qui est l'asymptote verticale à l'origine
- Résistance d'écoulement $R_M$ qui est l'asymptote oblique aux grandes intensités
- $a_0$ qui est l'ordonnée à l'origine de l'asymptote oblique.

En effet la courbe COT présente une asymptote verticale à l'origine et une asymptote oblique aussi appelée asymptote oblique aux grandes intensités.

Alternativement une étape d'acquisition d'une valeur de vitesse d'une ou plusieurs unités motrices musculaires d'un sujet v(t) et d'une valeur de débit d'oxygène $\varphi_{O2}$ (t) peut être remplacée par une étape de réalisations d'efforts (i) à l'aide d'un dispositif de test par le sujet et une étape de mesure (ii) instantanée de la vitesse de déplacement d'une ou plusieurs unités motrices musculaires du sujet v(t) et du débit d'oxygène $\varphi_{O2}$ (t). Alternativement, l'étape d'acquisition peut être précédée par une étape de mesure instantanée de la vitesse de déplacement d'une ou plusieurs unités motrices musculaires du sujet v(t), et du débit d'oxygène $\varphi O2$ (t) de ce sujet. En outre, ces valeurs peuvent avoir été mesurées de façon instantanée lors de la réalisation d'effort par un sujet réalisant un test d'effort ou une épreuve d'effort sur un dispositif de test.

**Selon d'autres caractéristiques optionnelles du procédé :**

- l'étape d'acquisition comporte également l'acquisition d'une puissance P(t) ou d'une force développée F(t),
- dans l'étape (iii), on trace une courbe Force F(t) en fonction de la vitesse v(t) d'une ou plusieurs unités motrices musculaires,
régie par l'équation suivante du modèle du muscle de Hill :

$$(F + a)(v + b) = c$$

- dans l'étape (iv), on détermine les paramètres métaboliques suivants à partir de cette courbe Force en fonction de la vitesse d'une ou plusieurs unités motrices musculaires :

- Force isométrique $F_{iso}$ correspondant à la force à vitesse nulle,
- Résistance de contre-réaction $R_{fb}$ correspondant à $F_{iso}/v_X$ avec $v_X$ égale à la vitesse à force nulle,
- Intensité métabolique de seuil $I_T$,

avec les équations suivantes :

1. $I_T = b$
2.

$$R_{fb} = \frac{a_0}{I_T} = \frac{a_0}{b}$$

3.

$$F_{iso} = \frac{c}{I_T} - a_0$$

Alternativement l'étape de mesure instantanée (ii) de la vitesse de déplacement d'une ou plusieurs unités motrices musculaires du sujet v(t), et du débit d'oxygène $\varphi_{O2}$ (t) peuvent être complétées par une étape d'acquisition d'une valeur de puissance P(t) et/ou de force développée F(t).

- dans l'étape (iv), on détermine les paramètres à différents instants

- on calcule le facteur de mérite $f_m$ égal à $f_{m=}$ ($Rf_{b*}$ $I_{T*}$ $F_{iso}$ ) / ($R_{M*}$ B)

- la vitesse de déplacement d'une ou plusieurs unités motrices musculaires a été mesurée à partir d'un dispositif de test correspondant à un tapis ou un vélo

- la vitesse de déplacement d'une ou plusieurs unités motrices musculaires v(t), et le débit d'oxygène $\varphi_{O2}$ (t) ont été obtenus lorsque que le sujet a été soumis à une charge variable $Z_{charge}$ (t) qui est une charge instantanée modulée temporellement ;

- l'étape d'acquisition en fonction du temps, comporte en outre l'acquisition d'une puissance développée P(t) ou d'une force F(t) ;

- dans l'étape de détermination de paramètres métaboliques d'une ou plusieurs unités motrices musculaires, on :

  - calcule des transformées de Laplace $\underline{F(p)}$ et $\underline{V(p)}$ à partir de la force F(t) et de la vitesse v(t);
  - calcule des impédances $\underline{Z(p)}=\underline{F(p)} / \underline{V(p)}$ ;
  - calcule à partir des impédances $\underline{Z(p)}$, les paramètres métaboliques suivants :

    ∘ Force isométrique $F_{iso}$,
    ∘ Une impédance de résistance de l'organisme $Z_{IM}$ ($\rho$) l'organisme étant représenté par la force isométrique et l'impédance de résistance de l'organisme de telle sorte que :

$$\underline{F(p)} = F_{iso} * \underline{Z(p)} / (\underline{Z_{IM}(p)} + \underline{Z(p)})$$

$$\underline{V(p)} = F_{iso} / (\underline{Z_{IM}(p)} + \underline{Z(p)})$$

  - calcule à partir de $\underline{Z_{IM}(p)}$ la partie réelle qui est le paramètre métabolique $R(I_M)$ puis les paramètres métaboliques suivants :

    ∘ Résistance de contre-réaction $R_{fb}$
    ∘ Intensité métabolique de seuil $I_T$
    ∘ Puissance basale B
    ∘ Résistance d'écoulement $R_M$

Alternativement, l'étape d'acquisition en fonction du temps peut correspondre ou être précédée par une étape de réalisation d'efforts, le sujet se déplaçant et étant soumis à une charge variable qui est une charge instantanée modulée dans le temps, ainsi qu'à une étape de mesure instantanée, mesurant la vitesse de déplacement d'une ou plusieurs assemblées d'unités motrices musculaires, de la puissance développée ou de la force et du débit d'oxygène.

- on modélise la puissance métabolique qui résulte de la différence entre les puissances entrantes et sortantes,

- on calcule la résistance mécanique totale de sortie qui s'écrit $R_{in} = R_M + R_{fb} = R_M + (Fi_{so} + a0) / (v_{T} + v)$,

   les grandeurs mesurées étant la force mécanique $F_M$ et la vitesse $v$, et

   avec la force $F_{iso} = F_B$ constante, on détermine $R_{in}$ en modulant la charge $R_L$

- le dispositif de test présente un freinage contrôlé en temps réel pour produire une modulation temporelle du freinage sur une plage de fréquences d'environ $10^{-2}$Hz à 10Hz, pour réaliser la charge $R_L$

- le freinage est réalisé par un moteur, couplé mécaniquement à une roue entraînée du dispositif de test, et qui agit en charge active.

- la forme des signaux de modulation temporelle est :

   - une sollicitation temporelle harmonique, à fréquence variable ;
   - une sollicitation impulsionnelle ;
   - une sollicitation indicielle.

Selon un autre mode de réalisation l'invention se rapporte à un dispositif de détermination de paramètres métaboliques d'une ou plusieurs unités motrices musculaires d'un sujet, comprenant :

- des moyens d'acquisition des efforts couplés à un dispositif de test, pour mesurer en fonction du temps : au moins la vitesse de déplacement d'une ou plusieurs unités motrices musculaires du sujet $v(t)$, et le débit d'oxygène $\varphi_{O2}(t)$ ;
- des moyens de calcul pour tracer une courbe COT entre le débit d'oxygène du sujet et la vitesse de déplacement d'une ou plusieurs unités motrices musculaires $v(t)$,

   la courbe COT étant couplée à la réponse musculaire par l'équation suivante :

$$COT = \frac{\Phi_-}{v} = a_0 + R_M v + \frac{B}{v}$$

   avec B la puissance basale d'une ou plusieurs unités motrices musculaires:
   $R_M$ la résistance d'écoulement d'une ou plusieurs unités motrices musculaires:
   ces moyens de calcul étant configurés pour déterminer des paramètres métaboliques suivants à partir de cette courbe :

   • Puissance basale B qui est l'asymptote verticale à l'origine
   • Résistance d'écoulement $R_M$ qui est l'asymptote oblique aux grandes intensités
   • $a_0$ qui est l'ordonnée à l'origine de l'asymptote oblique

Selon d'autres caractéristiques optionnelles du dispositif :

- les moyens d'acquisition sont configurés pour mesurer une puissance P(t) ou une force développée F(t),

- les moyens de calcul sont configurés pour tracer une courbe Force F(t) en fonction de la vitesse v(t) d'une ou plusieurs unités motrices musculaires, régie par l'équation suivante du modèle du muscle de Hill :

$$(F + a)(v + b) = c$$

- ces moyens de calcul sont configurés pour déterminer les paramètres métaboliques suivants à partir de cette courbe Force en fonction de la vitesse d'une ou plusieurs unités motrices musculaires:

  • Force isométrique $F_{iso}$ correspondant à la force à vitesse nulle,
  • Résistance de contre-réaction $R_{fb}$ correspondant à $F_{iso}/v_X$ avec $v_X$ égale à la vitesse à force nulle,
  • Intensité métabolique de seuil $I_T$,

Avec les équations suivantes :

1.

$$I_T = b$$

2.

$$R_{fb} = \frac{a_0}{I_T} = \frac{a_0}{b}$$

3.

$$F_{iso} = \frac{c}{I_T} - a_0$$

- les moyens de calcul sont configurés pour déterminer un facteur de mérite $f_m$ égal à

$$f_m = (R_{fb^*}\ I_{T^*}\ F_{iso}) / (R_{M^*}\ B)$$

- le dispositif de test est de type tapis ou vélo,

- les moyens d'acquisition sont configurés pour mesurer une puissance P(t) ou une force F(t),

- le dispositif de test des efforts est configuré pour soumettre le sujet à une charge variable $Z_{charge}$ (t) qui est une charge instantanée modulée temporellement ;

- les moyens de calcul sont configurés pour déterminer des paramètres métaboliques d'une ou plusieurs unités motrices musculaires, par :

  • un calcul des transformées de Laplace $\underline{F(p)}$ et $\underline{V(p)}$ à partir de la force F(t) et de la vitesse v(t);

  • un calcul des impédances $\underline{Z(p)} = \underline{F(p)} / \underline{V(p)}$ ;

  • un calcul à partir des impédances $\underline{Z(p)}$, des paramètres métaboliques suivants :

    ◦ Force isométrique $F_{iso}$,
    ◦ Une impédance de résistance de l'organisme $\underline{Z_{IM}\ (p)}$

  l'organisme étant représenté par la force isométrique et l'impédance de résistance de l'organisme de telle sorte que :

$$\underline{F(p)} = F_{iso}{}^* \underline{Z(p)} / (\underline{Z_{IM}\ (p)} + \underline{Z(p)})$$

$$\underline{V(p)} = F_{iso} / (\underline{Z_{IM}\ (p)} + \underline{Z(p)})$$

  • le calcul à partir de $\underline{Z_{IM}(p)}$ la partie réelle qui est le paramètre métabolique $R(I_M)$ puis les paramètres métaboliques suivants :

- ◦ Résistance de contre-réaction $R_{fb}$
- ◦ Intensité métabolique de seuil $I_T$
- ◦ Puissance basale B
- ◦ Résistance d'écoulement $R_M$

- le dispositif de test présente un frein contrôlé en temps réel pour produire une modulation temporelle du freinage sur une plage de fréquences d'environ $10^{-2}$Hz à 10Hz, par exemple pour réaliser une charge $R_L$

- le frein est réalisé par un moteur, couplé mécaniquement à une roue entraînée du dispositif de test, et qui agit en charge active.

## DESCRIPTION DES FIGURES

D'autres objectifs, caractéristiques et avantages sortiront de la description détaillée qui suit en référence aux dessins donnés à titre illustratif et non limitatif parmi lesquels :

- la figure 1 est un exemple de réponse force-vitesse (en haut), et puissance-vitesse (en bas) pour trois muscles différents : muscles lents (tirets), muscles moyens (tiret-point), muscles rapides (trait plein) ;
- la figure 2 représente un tracé de Φ- et du COT en fonction de l'intensité métabolique pour différents organismes ;
- les figures 3 représentent le principe de l'invention qui permet la détermination des paramètres métaboliques ;
- la figure 4 représente un schéma du dispositif selon l'invention ;
- la figure 5 représente la schématisation du bilan des puissances a), et du formalisme nodal associé b);
- la figure 6 représente une cartographie synthétique des paramètres métaboliques de deux individus ou sujets.

## DESCRIPTION DE L'INVENTION

La présente invention concerne la détermination de paramètres métaboliques intrinsèques et extrinsèques d'une ou plusieurs unités motrices musculaires constituées d'un ou de plusieurs muscles qui définissent une carte d'identité de l'état physique d'un sujet.
Cette détermination repose :

- Sur une modélisation thermodynamique qui définit les paramètres sans ambiguïté.
- Sur un dispositif de mesure à l'effort selon un protocole et une extraction de paramètres adéquats.

Chaque unité motrice musculaire est ainsi constituée d'un muscle, et les paramètres métaboliques sont déterminés en général sur plusieurs unités motrices considérées.
De préférence, le procédé selon l'invention est mis en oeuvre par au moins un moyen de calcul 3, tel qu'un processeur associé à une mémoire. Le ou les moyens de calcul 3 étant apte(s) à, de préférence configuré(s) pour, tracer une courbe COT entre le débit d'oxygène du sujet et la vitesse de déplacement du sujet v(t), et pour déterminer des paramètres métaboliques à partir de cette courbe. En outre, le ou les moyens de calcul 3 sont apte(s) à, de préférence configuré(s) pour, exécuter les différentes étapes d'un procédé selon l'invention.
En outre, on entend par « moyen d'acquisition » au sens de l'invention un dispositif de test et/ou des moyens de mesure.

### Résumé théorique

Le coeur de la modélisation repose sur la production de puissance métabolique par un organisme et est issue de la publication (Christophe Goupil et al 2019 New J. Phys. 21 023021 « Thermodynamics of metabolic energy conversion under muscle load »).
Cette puissance métabolique peut se décomposer en une force métabolique et une intensité métabolique, dont le produit des deux grandeurs donne la puissance métabolique.
Il est considéré que cette puissance métabolique est transmise, via les membres, sous forme de puissance mécanique et de vitesse de déplacement qui sont les grandeurs effectivement mesurées expérimentalement.
Le modèle thermodynamique développé est en parfait accord avec la modélisation phénoménologique de Hill, dont il fournit les fondations thermodynamiques.
La production de puissance métabolique résulte de la différence des puissances entrantes, Φ+, et sortantes, Φ-, de l'organisme.

Φ+ correspond à la fourniture en nutriment (principalement en glucose), tandis que Φ- est un flux de déchet qui comprend la chaleur excédentaire et surtout la production des métabolites réactionnels de type acide lactique.

Ce point est fondamental, car il en résulte que le flux Φ- peut être considéré, dans une large mesure, proportionnel au flux d'oxygène consommé par l'organisme.

Le modèle théorique conduit aux expressions suivantes :

$$\Phi_{+} = \frac{F_{\mathrm{iso}}I_M / \eta_{\mathrm{c}} + B}{I_T + I_M} I_T$$

$$\Phi_{-} = R_M I_M^2 + \frac{R_{\mathrm{fb}}I_M^2 + F_{\mathrm{iso}}\left(\frac{1}{\eta_{\mathrm{c}}} - 1\right)I_M + B}{I_T + I_M} I_T$$

Avec :

- $I_M$ : intensité métabolique (considérée proportionnelle à la vitesse de déplacement du membre qui, elle, est mesurée) ;
- $F_{iso}$ : force isométrique (extraite des courbes d'effort) ;
- $\eta_{\mathrm{c}}$: rendement thermodynamique maximal ;
- B : puissance basale (extraite des courbes d'effort) ;
- $I_T$ : intensité métabolique de seuil (extraite des courbes d'effort).

Ces notions ont été introduites dans la publication (Christophe Goupil et al 2019 New J. Phys.21 023021 « Thermodynamics of metabolic energy conversion under muscle load »).

La puissance métabolique résulte de la différence entre les puissances entrantes et sortantes, et s'écrit :

$$P = \Phi_{+} - \Phi_{-} = F_M I_M = \left[ F_{\mathrm{iso}} - \left( R_M + \frac{F_{\mathrm{iso}} + R_{\mathrm{fb}}I_T}{I_T + I_M} \right) I_M \right] I_M$$

La puissance métabolique fait intervenir deux termes supplémentaires par rapport aux précédents, il s'agit de $R_M$ et $R_{fb}$, avec :

- $R_M$ : résistance d'écoulement (extraite des courbes d'effort) aussi appelée résistance visqueuse;
- $R_{fb}$ : résistance de contre-réaction (extraite des courbes d'effort).

La paramétrisation en cinq paramètres contient donc à la fois des paramètres métaboliques ($I_T$, B) et mécaniques ($F_{iso}$, $R_M$). En ce qui concerne le terme $R_{fb}$ ce dernier est d'origine métabolique mais sa traduction est mécanique.

L'ensemble des paramètres peut se rassembler en un facteur de mérite métabolique qui est, en quelque sorte, le scalaire résumé de l'état de santé du sujet,

$$f_M = \frac{R_{\mathrm{fb}}F_{\mathrm{iso}}I_T}{R_M B}$$

L'ensemble des paramètres est maintenant établi, il reste à préciser certaines relations et méthodes expérimentales.

## Paramétrisation de Hill

Comme indiqué plus haut, le modèle métabolique a permis de donner les fondements thermodynamiques du modèle du muscle de Hill.

Hill propose une expression phénoménologique de la réponse force-vitesse d'une ou plusieurs unités motrices musculaires, selon :

$$(F + a)(v + b) = c$$

Avec :

- $F$ : force mécanique (mesurée) ;

- $v$ : vitesse de contraction (mesurée).

Le modèle théorique développé a permis d'obtenir les correspondances suivantes:

- $a = R_{fb}I_T + R_M I_M = a_0 + R_M I_M \approx a_0 = cte$
- $b = I_T = cte$
- $c = (F_{iso} + R_{fb}I_T) I_T = cte$

La courbe de réponse typique force-vitesse permet de remonter aux valeurs des trois paramètres, puisque la forme globale est définie par deux points extrémaux, $F_{iso}$ et la vitesse à force nulle $v_X \approx \frac{F_{iso}}{R_{fb}}$ .

Le creusement caractéristique de la courbe définit $I_T$ .

Sont donc extrait les paramètres suivants :

- $F_{iso}$

- $R_{fb}$

- $I_T$

## Bilan des paramètres

Nous avons cinq paramètres :

- $F_{iso}$ : force isométrique (extraite des courbes d'effort) ;
- $B$ : puissance basale (extraite des courbes d'effort) ;
- $I_T$ : intensité métabolique de seuil (extraite des courbes d'effort) ;
- $R_M$ : résistance d'écoulement (extraite des courbes d'effort) ;
- $R_{fb}$ : résistance de contre-réaction (extraite des courbes d'effort).

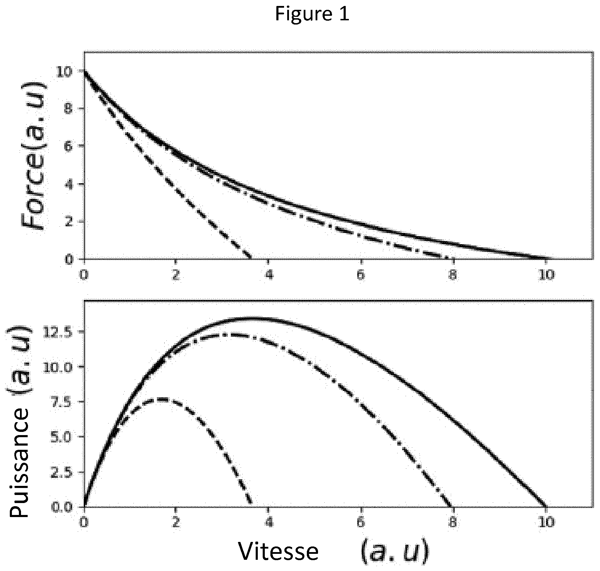

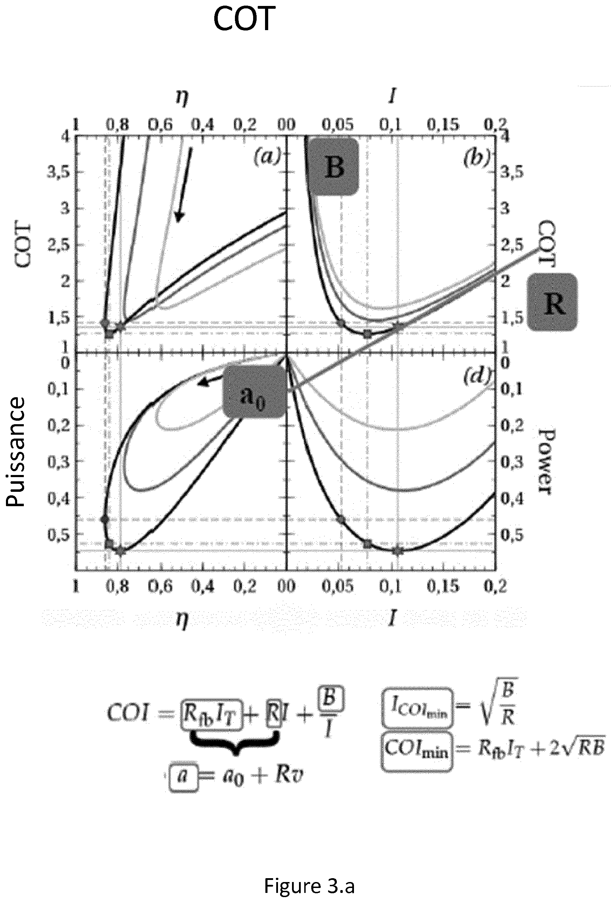

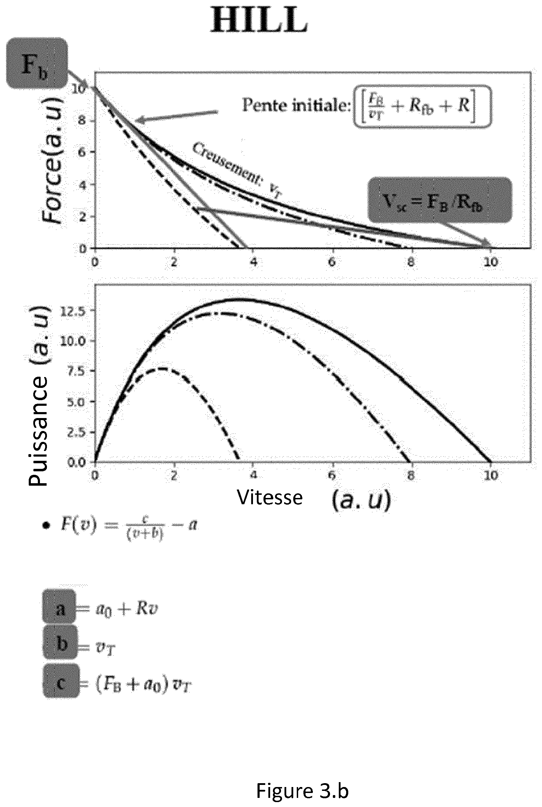

Et nous avons une expérience force-vitesse (figure 1 et figure 3) qui en détermine trois :

1. $I_T = b$
2.

$$R_{fb} = \frac{a_0}{I_T} = \frac{a_0}{b}$$

3.

$$F_{iso} = \frac{c}{I_T} - a_0$$

Il en reste deux à déterminer. C'est le rôle de la mesure de COT.

## COI et COT

En utilisant la proportionnalité entre le débit d'oxygène et $\Phi-$ , on peut extraire les termes manquants.

On définit le Cost of Oxygen Index (COI) par :

$$COI = \frac{\Phi_-}{I_M} = a_0 + R_M I_M + \frac{B}{I_M}$$

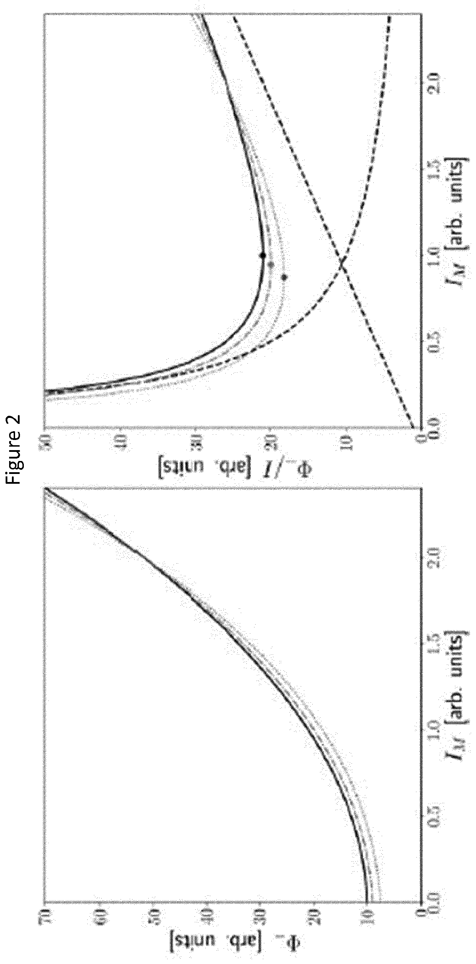

La forme de son tracé est représentée sur la figure 2.

On note que la courbe présente une asymptote verticale à l'origine, et une asymptote oblique aux grandes intensités. Ces deux asymptotes permettent d'extraire respectivement :

- $B$

- $R_M$

On note que l'ordonné à l'origine permet aussi de retrouver le paramètre $a_0$ de Hill. L'ensemble des cinq paramètres qui caractérisent l'organisme est donc accessible.

Dans le cas du déplacement, et en utilisant la proportionnalité entre $I_M$ et la vitesse, on peut définir le Cost of Oxygen Transport (COT), qui est une grandeur très largement utilisée dans la littérature.

En effet, du point de vue dimensionnel, le COT correspond à la quantité de déchet énergétique produit pour déplacer l'organisme sur une unité de longueur.

Son expression est :

$$COT = \frac{\Phi_-}{v} = a_0 + R_M v + \frac{B}{v}$$

La forme du COT est donc isomorphe à celle du COI.

Conclusion : Moyennant une épreuve d'effort mesurant les puissances, les vitesses et les débits d'oxygène, il est possible de mesurer les cinq paramètres métaboliques définissant la cartographie de l'état d'un sujet. En outre, le facteur de mérite permet de résumé la situation du sujet.

### Réalisation expérimentale

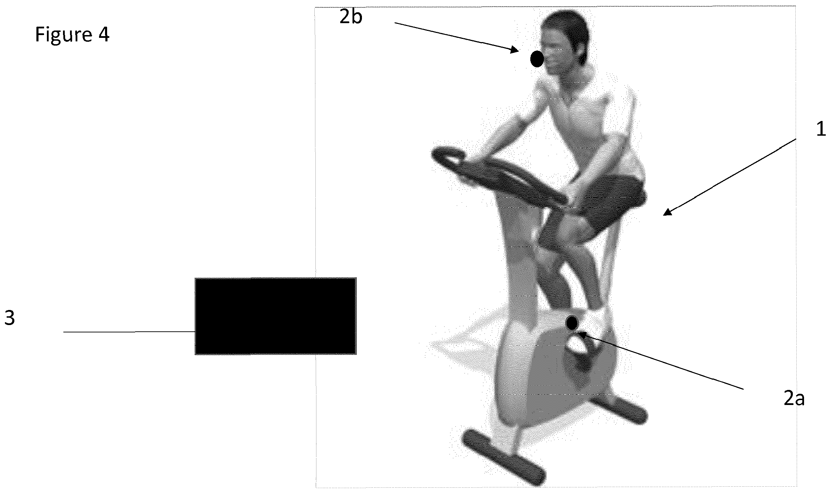

Du point de vue expérimental, il convient d'envisager un dispositif de test d'effort de type tapis ou vélo, tel qu'illustré sur la figure 4.

Les grandeurs à mesurer seront :

- Vitesse de marche ou de pédalage ;
- Puissance développée (La force ou le couple produit en seront extraits, connaissant la vitesse) ;
- Consommation d'oxygène.

### Protocole

#### Principe

Du point de vue pratique, les mesures reviennent à extraire l'impédance de sortie mécanique de l'organisme.

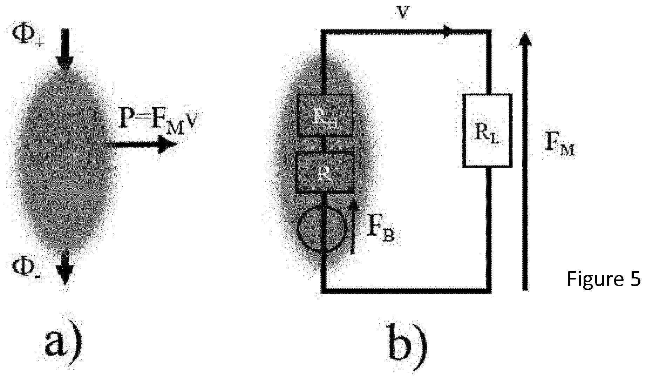

En effet, la schématisation équivalente selon une approche nodale est illustré sur la figure 5.

Le ou les membres, ou plus précisément la ou les unité(s) motrice(s) musculaire(s) agissante(s), est/sont assimilé(es) à une source de puissance mécanique délivrant une force, ici représentée par un générateur. En série avec ce générateur se trouvent les résistances $R_M$ et $R_{fb}$ qui induisent une perte de puissance par dissipation visqueuse.

La puissance mécanique effectivement disponible se situe en bout de cette chaîne. Cette puissance résulte du produit entre la force disponible en bout de chaîne, multipliée par la vitesse de déplacement des membres.

La résistance mécanique de sortie s'écrit $R_{in} = R + R_H(v) = R_M + (F_{ISO} + a_0)/(v_T + v)$

Sachant que les grandeurs mesurées sont la force mécanique $F_M$ et la vitesse $v$, d'une part, et que la force $F_{ISO} = F_B$ est constante, on peut, en modulant la charge $R_L$ obtenir l'extraction de la valeur de $R_{in}$. Selon le type de protocole envisagé, la charge peut être de type freinage empêchant le déplacement, ou encore, masse en opposition au déplacement, comme dans le cas des dispositifs en anglais dit « leg-press »).

Réalisation

L'homme de l'art comprendra que la réalisation expérimentale peut s'opérer par l'usage de différents dispositifs (tapis, vélo, dispositif en anglais dit « leg-press »), selon la détermination des paramètres métaboliques porte sur un membre, une partie, ou totalité de l'organisme.

Considérons le cas d'un dispositif de type vélo d'entraînement, comme illustré sur la figure 4. La charge $R_L$ est réalisée expérimentalement par un frein contrôlé en temps réel pour produire une modulation temporelle du freinage sur une plage de fréquences d'environ $10^{-2}$Hz - 10Hz. Le frein peut être réalisé par un moteur, couplé mécaniquement à la roue entraînée, et qui agit en charge active.

La procédure expérimentale est équivalente à une sollicitation harmonique telle qu'on les rencontre dans les mesures de spectroscopie d'impédance. L'analogie électrique de ce protocole mécanique est la technique de spectroscopie d'impédance utilisée en électrochimie pour déterminer les performances des batteries électriques.

La forme des signaux de modulation peut être de différents types, en suivant la tradition des analyses par spectroscopie d'impédance :

- sollicitation temporelle harmonique, à fréquence variable ;
- sollicitation impulsionnelle, cette sollicitation revient à brutalement changer le freinage et l'effacer immédiatement (déconseillé pour raison de risque de blessures) ;
- sollicitation indicielle, cette sollicitation revient à brutalement changer le freinage et le conserver (technique connue sous le nom en anglais de "Wingate"), mais son exploitation n'est en général pas faite au-delà de l'analyse de la forme des courbes).

Ainsi à l'issue du protocole réalisé sur l'appareillage, la cartographie synthétique se compose des éléments suivants :

- Puissance basale ;
- Vitesse de libération de l'énergie chimique ;
- Viscosité métabolique, statique et dynamique ;
- Viscosité mécanique, statique et dynamique ;
- Force isométrique.

Mesurées sous effort stationnaires, ces grandeurs ont des valeurs réelles.

En revanche, mesurés sous effort transitoire, les paramètres de viscosité présentent une partie imaginaire qui rend compte des termes élastiques et des termes inertiels.

L'ensemble de ces paramètres permet d'obtenir la valeur du facteur de mérite métabolique qui est l'indicateur principal de la potentialité de l'individu. A ce titre, l'appareillage proposé peut à la fois servir au suivi de la performance sportive, qu'à la rééducation fonctionnelle. Il permet d'obtenir une cartographie synthétique de la réponse à l'effort d'un individu, ce que ne permettent pas d'obtenir l'état de l'art dans lequel les mesures et les protocoles existants sont réalisés séparément.

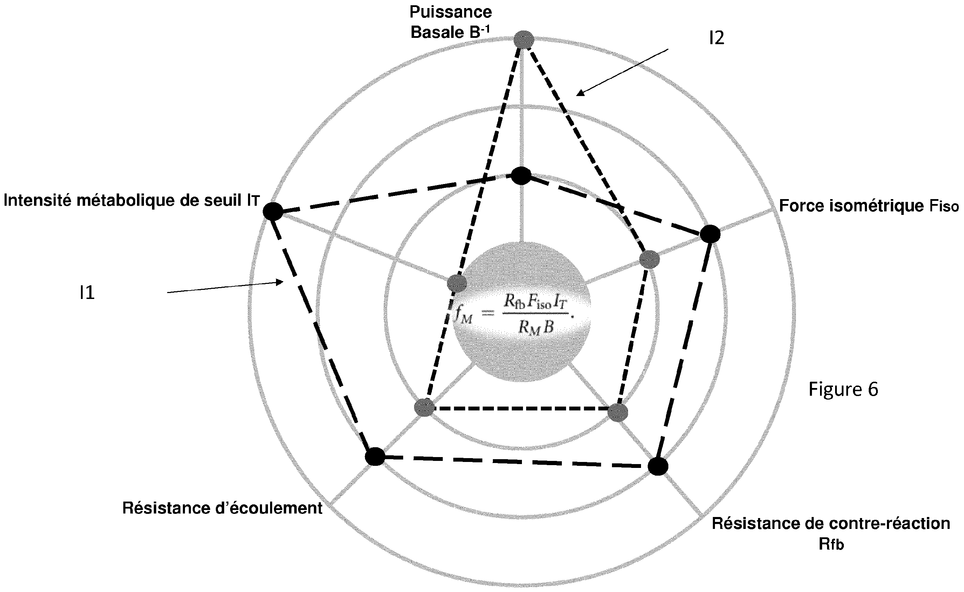

Comme représenté sur la figure 6, deux individus présentent différentes valeurs des paramètres métaboliques.

L'individu I1 présente une puissance basale assez faible, une force isométrique et une intensité métabolique de seuil élevées. Ces deux dernières grandeurs montrent que l'individu I1 est capable de soumettre son organisme à un effort physique conséquent. Sa faible puissance basale caractérise une dominante de muscle de type lents. Il s'agit donc d'un individu adapté à un effort de type endurant, plutôt qu'explosif. Les valeurs des résistances découlement et de contre-réaction assez élevées montrent qu'il existe une marge de progression à la fois physique, par l'entrainement (baisse de la résistance d'écoulement), et un progression éventuelle métabolique, (baisse de la résistance de contre-réaction) par une meilleure métabolisation des nutriments.

L'individu I2 présente une puissance basale importante, une intensité de seuil faible ainsi qu'une force isométrique réduite. Les résistances sont elles aussi de faible valeur. Dans ce cas il s'agit d'un individu présentant à priori un très bon potentiel pour l'effort explosif (puissance basale importante), mais qui se trouve fortement réduit par la faiblesse de l'intensité seuil. Cette contradiction peut s'expliquer par la présence d'une blessure qui ne permet pas à un organisme normalement performant d'exprimer la totalité de son potentiel. On peut donc observer que la collection des cinq paramètres permet de distinguer la performance mesurée des potentialités d'un individu.

**Revendications**

**1.** Procédé de détermination de paramètres métaboliques d'une ou plusieurs unités motrices musculaires d'un sujet par couplage de sa réponse musculaire à sa réponse ventilatoire, comprenant les étapes suivantes :

(i)+(ii)-une étape d'acquisition, par des moyens d'acquisition (2a, 2b), en fonction du temps d'une vitesse de déplacement d'une ou plusieurs unités motrices musculaires d'un sujet v(t), et d'un débit d'oxygène $\varphi_{O2}$ (t) du sujet ;
(iii)-une étape de tracé, par des moyens de calcul (3), de la courbe COT entre le débit d'oxygène du sujet et la vitesse de déplacement d'une ou plusieurs unités motrices musculaires v(t) du sujet,

la courbe COT étant couplée à la réponse musculaire par l'équation suivante :

$$COT = \frac{\Phi_-}{v} = a_0 + R_M v + \frac{B}{v}$$

avec
B la puissance basale d'une ou plusieurs unités motrices musculaires $R_M$ la résistance d'écoulement d'une ou plusieurs unités motrices musculaires ;

(iv)-une étape de détermination, par les moyens de calcul (3), des paramètres métaboliques suivants à partir de cette courbe COT :

- Puissance basale B qui est l'asymptote verticale à l'origine ;
- Résistance d'écoulement $R_M$ qui est l'asymptote oblique;
- $a_0$ qui est l'ordonnée à l'origine de l'asymptote oblique.

2. Procédé selon la revendication précédente, dans lequel :

- l'étape d'acquisition comporte également l'acquisition d'une puissance P(t) ou d'une force développée F(t),
- dans l'étape (iii), on trace une courbe Force F(t) en fonction de la vitesse v(t) d'une ou plusieurs unités motrices musculaires,
régie par l'équation suivante du modèle du muscle de Hill :

$$(F + a)(v + b) = c$$

- dans l'étape (iv), on détermine les paramètres métaboliques suivants à partir de cette courbe Force en fonction de la vitesse d'une ou plusieurs unités motrices musculaires :

- Force isométrique $F_{iso}$ correspondant à la force à vitesse nulle,
- Résistance de contre-réaction $R_{fb}$ correspondant à $F_{iso}/v_X$ avec vx égale à la vitesse à force nulle,
- Intensité métabolique de seuil $I_T$,

avec les équations suivantes :

1. $I_T = b$
2.

$$R_{fb} = \frac{a_0}{I_T} = \frac{a_0}{b}$$

3.

$$F_{iso} = \frac{c}{I_T} - a_0 \quad .$$

3. Procédé selon l'une des revendications précédentes, dans lequel, dans l'étape (iv), on détermine les paramètres à différents instants.

4. Procédé selon l'une des revendications 2 à 3, dans lequel on calcule le facteur de mérite $f_m$ égal à $f_m = (Rf_{b*} I_{T*} F_{iso})$ / $(R_{M*} B)$.

5. Procédé selon l'une des revendications précédentes, dans lequel la vitesse de déplacement d'une ou plusieurs unités motrices musculaires a été mesurée à partir d'un dispositif de test (1) correspondant à un tapis ou un vélo.

6. Procédé selon l'une des revendications précédentes, dans lequel :

   - la vitesse de déplacement d'une ou plusieurs unités motrices musculaires $v(t)$, et le débit d'oxygène $\varphi_{O2}(t)$ ont été obtenus lorsque que le sujet a été soumis à une charge variable $Z_{charge}(t)$ qui est une charge instantanée modulée temporellement ;
   - l'étape d'acquisition en fonction du temps, comporte en outre l'acquisition d'une puissance développée $P(t)$ ou d'une force $F(t)$ ;
   - dans l'étape de détermination de paramètres métaboliques d'une ou plusieurs unités motrices musculaires, on :

     • calcule des transformées de Laplace $F(p)$ et $V(p)$ à partir de la force $F(t)$ et de la vitesse $v(t)$;
     • calcule des impédances $Z(p)=F(p) / V(p)$ ;
     • calcule à partir des impédances $Z(p)$, les paramètres métaboliques suivants :

       ○ Force isométrique $F_{iso}$,
       ○ Une impédance de résistance de l'organisme $Z_{IM}(p)$

     l'organisme étant représenté par la force isométrique et l'impédance de résistance de l'organisme de telle sorte que :

$$F(p)= F_{iso}{}^* Z(p) / (Z_{IM}(p) + Z(p))$$

$$V(p) = F_{iso} / (Z_{IM}(p) + Z(p))$$

     • calcule à partir de $Z_{IM}(p)$ la partie réelle qui est le paramètre métabolique $R(I_M)$ puis les paramètres métaboliques suivants :

       ○ Résistance de contre-réaction $R_{fb}$,
       ○ Intensité métabolique de seuil $I_T$,
       ○ Puissance basale B,
       ○ Résistance d'écoulement $R_M$.

7. Procédé selon l'une des revendications précédentes, dans lequel :

   - on modélise la puissance métabolique qui résulte de la différence entre les puissances entrantes et sortantes,
   - on calcule la résistance mécanique totale de sortie qui s'écrit $R_{in} = R_M + R_{fb} = R_M + (Fi_{so}+a0) / (v_T+v)$,
   les grandeurs mesurés étant la force mécanique $F_M$ et la vitesse $v$, et
   avec la force $F_{iso} = F_B$ constante, on détermine $R_{in}$ en modulant la charge $R_L$.

8. Procédé selon la revendication 5 et la revendication précédente, dans lequel le dispositif de test présente un freinage contrôlé en temps réel pour produire une modulation temporelle du freinage sur une plage de fréquences d'environ $10^{-2}$ Hz à 10 Hz, pour réaliser la charge $R_L$.

9. Procédé selon la revendication précédente, dans lequel le freinage est réalisé par un moteur, couplé mécaniquement à une roue entraînée du dispositif de test, et qui agit en charge active.

10. Procédé selon la revendication précédente, dans lequel la forme des signaux de modulation temporelle est :

   - une sollicitation temporelle harmonique, à fréquence variable ;

- une sollicitation impulsionnelle ;
- une sollicitation indicielle.

**11.** Dispositif de détermination de paramètres métaboliques d'une ou plusieurs unités motrices musculaires d'un sujet, comprenant :

- des moyens d'acquisition (2a, 2b) des efforts couplés à un dispositif de test (1), pour mesurer en fonction du temps : au moins la vitesse de déplacement d'une ou plusieurs unités motrices musculaires du sujet v(t), et le débit d'oxygène $\varphi_{O2}$ (t) ;
- des moyens de calcul (3) pour tracer une courbe COT entre le débit d'oxygène du sujet et la vitesse de déplacement d'une ou plusieurs unités motrices musculaires v(t),
la courbe COT étant couplée à la réponse musculaire par l'équation suivante :

$$COT = \frac{\Phi_-}{v} = a_0 + R_M v + \frac{B}{v}$$

avec B la puissance basale d'une ou plusieurs unités motrices musculaires:
$R_M$ la résistance d'écoulement d'une ou plusieurs unités motrices musculaires:
ces moyens de calcul (3) étant configurés pour déterminer des paramètres métaboliques suivants à partir de cette courbe COT :

• Puissance basale B qui est l'asymptote verticale à l'origine ;
• Résistance d'écoulement $R_M$ qui est l'asymptote oblique ;
• $a_0$ qui est l'ordonnée à l'origine de l'asymptote oblique.

**12.** Dispositif selon la revendication 11, dans lequel :

- les moyens d'acquisition (2a, 2b) sont configurés pour mesurer une puissance P(t) ou une force développée F(t),
- les moyens de calcul (3) sont configurés pour tracer une courbe Force F(t) en fonction de la vitesse v(t) d'une ou plusieurs unités motrices musculaires,
régie par l'équation suivante du modèle du muscle de Hill :

$$(F + a)(v + b) = c$$

- ces moyens de calcul (3) sont configurés pour déterminer les paramètres métaboliques suivants à partir de cette courbe Force en fonction de la vitesse d'une ou plusieurs unités motrices musculaires :

• Force isométrique $F_{iso}$ correspondant à la force à vitesse nulle,
• Résistance de contre-réaction $R_{fb}$ correspondant à $F_{iso}/v_X$ avec vx égale à la vitesse à force nulle,
• Intensité métabolique de seuil $I_T$,

Avec les équations suivantes :

1. $I_T = b$
2.

$$R_{fb} = \frac{a_0}{I_T} = \frac{a_0}{b}$$

3.

$$F_{iso} = \frac{c}{I_T} - a_0$$ .

**13.** Dispositif selon l'une des revendications 11 ou 12, dans lequel les moyens de calcul (3) sont configurés pour déterminer un facteur de mérite $f_m$ égal à $f_m = (Rf_{b*} I_{T*} F_{iso}) / (R_{M*} B)$.

14. Dispositif selon l'une des revendications 11 à 13, dans lequel le dispositif de test (1) est de type tapis ou vélo.

15. Dispositif selon l'une des revendications 11 à 14, dans lequel :

- les moyens d'acquisition (2a, 2b) sont configurés pour mesurer une puissance P(t) ou une force F(t),
- le dispositif de test (1) des efforts est configuré pour soumettre le sujet à une charge variable $Z_{charge}$ (t) qui est une charge instantanée modulée temporellement ;
- les moyens de calcul (3) sont configurés pour déterminer des paramètres métaboliques d'une ou plusieurs unités motrices musculaires, par :

• un calcul des transformées de Laplace F(p) et V(p) à partir de la force F(t) et de la vitesse v(t);
• un calcul des impédances Z(p)=F(p) / V(p) ;
• un calcul à partir des impédances Z(p), des paramètres métaboliques suivants :

◦ Force isométrique $F_{iso}$,
◦ Une impédance de résistance de l'organisme $Z_{IM}$ (p)

l'organisme étant représenté par la force isométrique et l'impédance de résistance de l'organisme de telle sorte que :

$$F(p) = F_{iso} * Z(p) / (Z_{IM} (p) + Z(p))$$

$$V(p) = F_{iso} / (Z_{IM} (p) + Z(p))$$

• le calcul à partir de $Z_{IM}(p)$ la partie réelle qui est le paramètre métabolique $R(I_M)$ puis les paramètres métaboliques suivants :

◦ Résistance de contre-réaction $R_{fb}$ ;
◦ Intensité métabolique de seuil $I_T$ ;
◦ Puissance basale B ;
◦ Résistance d'écoulement $R_M$.

16. Dispositif selon la revendication précédente, dans lequel le dispositif de test présente un frein contrôlé en temps réel pour produire une modulation temporelle du freinage sur une plage de fréquences d'environ $10^{-2}$Hz à 10Hz.

17. Dispositif selon la revendication précédente, dans lequel le frein est réalisé par un moteur, couplé mécaniquement à une roue entraînée du dispositif de test, et qui agit en charge active.

**Patentansprüche**

1. Verfahren zur Bestimmung von Stoffwechselparametern von einer oder mehreren motorischen Einheiten von Muskeln eines Probanden durch Kopplung seiner Muskelreaktion mit seiner ventilatorischen Reaktion, umfassend die folgenden Schritte:

(i)+(ii)- einen Schritt eines Erfassens einer Geschwindigkeit einer Verlagerung von einer oder mehreren motorischen Einheiten von Muskeln eines Probanden v(t) in Abhängigkeit von der Zeit und eines Sauerstoffdurchsatzes $\varphi_{O2}$ (t) des Probanden durch Erfassungsmittel (2a, 2b);
(iii)- einen Schritt eines Zeichnens der Kurve COT zwischen dem Sauerstoffdurchsatz des Probanden und der Geschwindigkeit der Verlagerung von einer oder mehreren motorischen Einheiten von Muskeln v(t) des Probanden durch Berechnungsmittel (3),
wobei die Kurve COT durch die folgende Gleichung mit der Muskelreaktion gekoppelt wird:

$$\mathrm{COT} = \frac{\Phi}{\upsilon} = a_0 + R_M \upsilon + \frac{B}{\upsilon}$$

wobei

B die basale Leistung von einer oder mehreren motorischen Einheiten von Muskeln ist;
$R_M$ der Strömungswiderstand von einer oder mehreren motorischen Einheiten von Muskeln ist;

(iv)- einen Schritt eines Bestimmens der folgenden Stoffwechselparameter aus dieser Kurve COT durch die Berechnungsmittel (3):

• der basalen Leistung B, die die vertikale Asymptote zu dem Ursprung ist;
• des Strömungswiderstands $R_M$, der die schiefe Asymptote ist;
• $a_0$, die die Ordinate zum Ursprung der schiefen Asymptote ist.

2. Verfahren nach dem vorhergehenden Anspruch, wobei:

- der Schritt des Erfassens außerdem das Erfassen einer Leistung P(t) oder einer entwickelten Kraft F(t) umfasst,
- im Schritt (iii) eine Kurve Kraft F(t) in Abhängigkeit von der Geschwindigkeit v(t) von einer oder mehreren motorischen Einheiten von Muskeln gezeichnet wird,
geregelt durch die folgende Gleichung des Hill-Muskelmodells:

$$(F + a)(\upsilon + b) = c$$

- im Schritt (iv) die folgenden Stoffwechselparameter aus dieser Kurve Kraft in Abhängigkeit von der Geschwindigkeit von einer oder mehreren motorischen Einheiten von Muskeln bestimmt werden:

• die isometrische Kraft $F_{iso}$, die der Kraft bei Nullgeschwindigkeit entspricht,
• der Gegenreaktionswiderstand $R_{fb}$, der $F_{iso}$/vx entspricht, wobei vx gleich der Geschwindigkeit bei Nullkraft ist;
• die Grenzstoffwechselintensität $I_T$,

mit den folgenden Gleichungen:

1. $I_T = b$
2.

$$R_{fb} = \frac{a_0}{I_T} = \frac{a_0}{b}$$

3.

$$F_{iso} = \frac{c}{I_T} - a_0 .$$

3. Verfahren nach einem der vorhergehenden Ansprüche, wobei im Schritt (iv) die Parameter zu unterschiedlichen Zeitpunkten bestimmt werden.

4. Verfahren nach einem der Ansprüche 2 bis 3, wobei die Gütezahl $f_m$ berechnet wird, die gleich $f_m = (R_{fb}*I_T*F_{iso}) / (R_M*B)$ ist.

5. Verfahren nach einem der vorhergehenden Ansprüche, wobei die Geschwindigkeit der Verlagerung von einer oder mehreren motorischen Einheiten von Muskeln anhand einer Testvorrichtung (1) gemessen wurde, die einem Laufband oder einem Fahrrad entspricht.

**6.** Verfahren nach einem der vorhergehenden Ansprüche, wobei:

- die Geschwindigkeit der Verlagerung von einer oder mehreren motorischen Einheiten von Muskeln v(t) und der Sauerstoffdurchsatz $\varphi_{O2}$ (t) erhalten werden, wenn der Proband einer variablen Belastung $Z_{Belastung}$ (t) unterzogen wurde, die eine zeitlich modulierte unmittelbare Belastung ist;
- der Schritt des Erfassens in Abhängigkeit von der Zeit weiterhin das Erfassen einer entwickelten Leistung P(t) oder einer Kraft F(t) umfasst;
- im Schritt des Bestimmens von Stoffwechselparametern von einer oder mehreren motorischen Einheiten von Muskeln:

   • Laplace-Transformierte F(p) und V(p) aus der Kraft F(t) und der Geschwindigkeit v(t) berechnet werden;
   • Impedanzen Z(p)=F(p) / V(p) berechnet werden;
   • die folgenden Stoffwechselparameter aus Impedanzen Z(p) berechnet werden:

      ∘ die isometrische Kraft $F_{iso}$;
      ∘ eine Widerstandsimpedanz des Organismus $Z_{IM}$(p),

   wobei der Organismus durch die isometrische Kraft und die Widerstandsimpedanz des Organismus derart dargestellt wird, dass:

$$F(p) = F_{iso} * Z(p) / (Z_{IM}(p) + Z(p))$$

$$V(p) = F_{iso} / (Z_{IM}(p) + Z(p))$$

   • aus $Z_{IM}$(p) der Realteil, der der Stoffwechselparameter $R(I_M)$ ist, und dann die folgenden Stoffwechselparameter berechnet werden:

      oder Gegenreaktionswiderstand $R_{fb}$,
      ∘ die Grenzstoffwechselintensität $I_T$,
      ∘ die basale Leistung B,
      oder Strömungswiderstand $R_M$.

**7.** Verfahren nach einem der vorhergehenden Ansprüche, wobei:

- ein Modell der Stoffwechselleistung, die aus der Differenz zwischen der Eingangs- und der Ausgangsleistung resultiert, erstellt wird,
- der mechanische Gesamtausgangswiderstand berechnet wird, der als $R_{in} = R_M + R_{fb} = R_M + (F_{iso} + a0) / (v_T + v)$ geschrieben wird,

   wobei die gemessenen Größen die mechanische Kraft $F_M$ und die Geschwindigkeit v sind, und wenn die Kraft $F_{iso} = F_B$ konstant ist, $R_{in}$ durch Modulieren der Belastung $R_L$ bestimmt wird.

**8.** Verfahren nach Anspruch 5 und dem vorhergehenden Anspruch, wobei die Testvorrichtung eine kontrollierte Bremsung in Echtzeit aufweist, um eine zeitliche Modulierung der Bremsung in einem Frequenzbereich von ungefähr $10^{-2}$ Hz bis 10 Hz zu produzieren, um die Belastung $R_L$ umzusetzen.

**9.** Verfahren nach dem vorhergehenden Anspruch, wobei die Bremsung durch einen Motor umgesetzt wird, der mechanisch an ein Abtriebsrad der Testvorrichtung gekoppelt ist, und die auf eine aktive Belastung einwirkt.

**10.** Verfahren nach dem vorhergehenden Anspruch, wobei die Form von Signalen einer zeitlichen Modulierung:

- eine zeitliche Schwingungsbeanspruchung mit variabler Frequenz;
- eine Impulsbeanspruchung;
- eine Indexbeanspruchung ist.

**11.** Vorrichtung zur Bestimmung von Stoffwechselparametern von einer oder mehreren motorischen Einheiten von Muskeln eines Probanden, umfassend:

- Mittel zur Erfassung (2a, 2b) von Anstrengungen, die an eine Testvorrichtung (1) gekoppelt sind, zum Messen in Abhängigkeit von der Zeit von: mindestens der Geschwindigkeit einer Verlagerung von einer oder mehreren motorischen Einheiten von Muskeln des Probanden v(t) und des Sauerstoffdurchsatzes $\varphi_{O2}$ (t) ;
- Berechnungsmittel (3) zum Zeichnen einer Kurve COT zwischen dem Sauerstoffdurchsatz des Probanden und der Geschwindigkeit der Verlagerung von einer oder mehreren motorischen Einheiten von Muskeln v(t), wobei die Kurve COT durch die folgende Gleichung mit der Muskelreaktion gekoppelt wird:

$$COT = \frac{\Phi_-}{v} = a_0 + R_M v + \frac{B}{v}$$

wobei

B die basale Leistung von einer oder mehreren motorischen Einheiten von Muskeln ist;
$R_M$ der Strömungswiderstand von einer oder mehreren motorischen Einheiten von Muskeln ist;
wobei diese Berechnungsmittel (3) zum Bestimmen der folgenden Stoffwechselparameter aus dieser Kurve COT konfiguriert sind:

• der basalen Leistung B, die die vertikale Asymptote zu dem Ursprung ist;
• des Strömungswiderstands $R_M$, der die schiefe Asymptote ist;
• $a_0$, die die Ordinate zum Ursprung der schiefen Asymptote ist.

**12.** Vorrichtung nach Anspruch 11, wobei:

- die Erfassungsmittel (2a, 2b) zum Messen einer Leistung P(t) oder einer entwickelten Kraft F(t) konfiguriert sind,
- die Berechnungsmittel (3) zum Zeichnen einer Kurve Kraft F(t) in Abhängigkeit von der Geschwindigkeit v(t) von einer oder mehreren motorischen Einheiten von Muskeln konfiguriert sind,
geregelt durch die folgende Gleichung des Hill-Muskelmodells:

$$(F + a)(v + b) = c$$

- diese Berechnungsmittel (3) zum Bestimmen der folgenden Stoffwechselparameter aus dieser Kurve Kraft in Abhängigkeit von der Geschwindigkeit von einer oder mehreren motorischen Einheiten von Muskeln konfiguriert sind:

• der isometrischen Kraft $F_{iso}$, die der Kraft bei Nullgeschwindigkeit entspricht,
• des Gegenreaktionswiderstands $R_{fb}$, der $F_{iso}/vx$ entspricht, wobei vx gleich der Geschwindigkeit bei Nullkraft ist;
• der Grenzstoffwechselintensität $I_T$,

mit den folgenden Gleichungen:

1. $I_T = b$
2.

$$R_{fb} = \frac{a_0}{I_T} = \frac{a_0}{b}$$

3.

$$F_{iso} = \frac{c}{I_T} - a_0 .$$

**13.** Vorrichtung nach einem der Ansprüche 11 oder 12, wobei die Berechnungsmittel (3) zum Bestimmen einer Gütezahl $f_m$ konfiguriert sind, die gleich $f_m = (R_{fb}*I_T*F_{iso}) / (R_M*B)$ ist.

**14.** Vorrichtung nach einem der Ansprüche 11 bis 13, wobei die Testvorrichtung (1) vom Laufband- oder Fahrrad-Typ ist.

**15.** Vorrichtung nach einem der Ansprüche 11 bis 14, wobei:

- die Erfassungsmittel (2a, 2b) zum Messen einer Leistung P(t) oder einer Kraft F(t) konfiguriert sind,
- die Testvorrichtung (1) für Anstrengungen zum Unterziehen des Probanden einer variablen Belastung $Z_{Belastung}$(t) konfiguriert ist, die eine zeitlich modulierte unmittelbare Belastung ist;
- die Berechnungsmittel (3) zum Bestimmen von Stoffwechselparametern von einer oder mehreren motorischen Einheiten von Muskeln konfiguriert sind:

• einer Berechnung von Laplace-Transformierten F(p) und V(p) aus der Kraft F(t) und der Geschwindigkeit v(t);
• einer Berechnung von Impedanzen Z(p) = F(p) / V(p);
• einer Berechnung der folgenden Stoffwechselparameter aus Impedanzen Z(p):

∘ der isometrischen Kraft $F_{iso}$,
∘ einer Widerstandsimpedanz des Organismus $Z_{IM}(p)$,

wobei der Organismus durch die isometrische Kraft und die Widerstandsimpedanz des Organismus derart dargestellt wird, dass:

$$F(p) = F_{iso}*Z(p) / (Z_{IM}(p) + Z(p))$$

$$V(p) = F_{iso} / (Z_{IM}(p) + Z(p))$$

• der Berechnung aus $Z_{IM}(p)$ des Realteils, der der Stoffwechselparameter $R(I_M)$ ist, und dann der folgenden Stoffwechselparameter:

∘ des Gegenreaktionswiderstands $R_{fb}$,
oder Grenzstoffwechselintensität $I_T$,
∘ der basalen Leistung B,
∘ des Strömungswiderstands $R_M$.

**16.** Vorrichtung nach dem vorhergehenden Anspruch, wobei die Testvorrichtung ein kontrolliertes Bremsen in Echtzeit aufweist, um eine zeitliche Modulierung der Bremsung in einem Frequenzbereich von ungefähr $10^{-2}$ Hz bis 10 Hz zu produzieren.

**17.** Vorrichtung nach dem vorhergehenden Anspruch, wobei das Bremsen durch einen Motor umgesetzt wird, der mechanisch an ein Abtriebsrad der Testvorrichtung gekoppelt ist, und das auf eine aktive Belastung einwirkt.

**Claims**

**1.** A method for determining the metabolic parameters of one or more of a subject's muscle motor units by coupling their muscular response to their ventilatory response, comprising the following steps:

(i)+(ii)- step of acquiring, by acquisition means (2a, 2b) as a function of time of a movement velocity of one or more of a subject's muscle motor units v(t), and of a subject's oxygen flow rate $\varphi_{O2}(t)$;
(iii)- a step of drawing, by calculation means (3), the COT curve between the subject's oxygen flow rate and the movement velocity of one or more of the subject's muscle motor units v(t),
the COT curve being coupled to the muscular response by the following equation:

$$COT = \frac{\Phi_-}{v} = a_0 + R_M v + \frac{B}{v}$$

with

> B the basal power of one or more muscle motor units;
> $R_M$ the dissipative metabolic resistance of one or more muscle motor units;

(iv)- a step of determining, by calculation means (3), the following metabolic parameters from this COT curve:

> • Basal power B which is the vertical asymptote at the origin;
> • Dissipative metabolic resistance $R_M$ which is the oblique asymptote;
> • $a_0$ which is the intercept of the oblique asymptote.

**2.** The method according to the preceding claim, wherein:

> - the acquisition step also comprises the acquisition of a power P(t) or a developed force F(t),
> - in step (iii), a Force curve F(t) is drawn as a function of the velocity v(t) of one or more muscle motor units, governed by the following equation from Hill's muscle model:

$$(F + a)(v + b) = c$$

> - in step (iv), the following metabolic parameters are determined from this Force curve as a function of the velocity of one or more muscle motor units:

> • Isometric force $F_{iso}$ corresponding to the force at zero velocity,
> • Feedback resistance $R_{fb}$ corresponding to $F_{iso}/v_x$ with $v_x$ equal to the velocity at zero force,
> • Threshold metabolic intensity $I_T$,

with the following equations:

1. $I_T = b$
2.

$$R_{fb} = \frac{a_0}{I_T} = \frac{a_0}{b}$$

3.

$$F_{iso} = \frac{c}{I_T} - a_0 \,.$$

**3.** The method according to one of the preceding claims wherein, in step (iv), the parameters are determined at different instants.

**4.** The method according to one of claims 2 to 3, wherein the figure of merit $f_m$ is calculated equal to $f_m = (R_{fb}*I_T*F_{iso}) / (R_M*B)$.

**5.** The method according to one of the preceding claims, wherein the movement velocity of one or more muscle motor units has been measured from a test device (1) corresponding to a treadmill or bike.

**6.** The method according to any one of the preceding claims, wherein:

> - the movement velocity of one more muscle motor units v(t), and the oxygen flow rate $\varphi_{O2}(t)$ have been obtained when the subject was subjected to a variable load $Z_{load}$ (t) which is a time-modulated instantaneous load;
- the acquisition step as a function of time also comprises the acquisition of a developed power P(t) or a force F(t),
- in the step of determining the metabolic parameters of one or more muscle motor units:

• the Laplace transforms F(p) and V(p) are calculated from the force F(t) and the velocity v(t);
• impedances Z(p)=F(p) / V(p) are calculated;
• from impedances Z(p), the following metabolic parameters are calculated:

    ○ Isometric force $F_{iso}$,
    ○ Resistance impedance of the body $Z_{IM}(p)$

the body being represented by the isometric force and the resistance impedance of the body so that:

$$F(p) = F_{iso} * Z(p) / (Z_{IM}(p) + Z(p))$$

$$V(p) = F_{iso} / (Z_{IM}(p) + Z(p))$$

• the real part which is the metabolic parameter $R(I_M)$ is calculated from $Z_{IM}(p)$ and then the following metabolic parameters are calculated:

    ○ Feedback resistance $R_{fb}$,
    ○ Threshold metabolic intensity $I_T$,
    ○ Basal power B,
    ○ Dissipative metabolic resistance $R_M$.

7. The method according to any one of the preceding claims, wherein:

- the metabolic power which results from the difference between the incoming and outgoing powers is modeled,
- the total output mechanical resistance is calculated, which is written $R_{in} = R_M + R_{fb} = R_M + (F_{iso} + a_0) / (V_T + V)$, the quantities measured being the mechanical force $F_M$ and the velocity v, and with the force $F_{iso} = F_B$ constant, $R_{in}$ is determined by modulating the load $R_L$.

8. The method according to claim 5 and the preceding claim, wherein the test device has real-time controlled braking to produce time-modulated braking over a frequency range of approximately $10^{-2}$ Hz to 10 Hz, to achieve the load $R_L$.

9. The method according to the preceding claim, wherein the braking is produced by a motor, mechanically coupled to a driven wheel of the test device, and which acts as an active load.

10. The method according to the preceding claim, wherein the form of the temporal modulation signals is:

- a harmonic temporal load, at variable frequency;
- a pulse load;
- an index load.

11. A device for determining the metabolic parameters of one or more of a subject's muscle motor units, comprising:

- force acquisition means (2a, 2b) coupled to a test device (1), to measure as a function of time: at least the movement velocity of one or more of the subject's muscle motor units v(t) and the oxygen flow rate $\varphi_{O2}(t)$;
- calculation means (3), to draw a COT curve between the subject's oxygen flow rate and the movement velocity of one or more of the muscle motor units v(t),

the COT curve being coupled to the muscular response by the following equation:

$$COT = \frac{\Phi_-}{v} = a_0 + R_M v + \frac{B}{v}$$

with B the basal power of one or more muscle motor units;
$R_M$ the dissipative metabolic resistance of one or more muscle motor units;
these calculation means (3) being configured to determine the following metabolic parameters from this COT curve:

- Basal power B which is the vertical asymptote at the origin;
- Dissipative metabolic resistance $R_M$ which is the oblique asymptote;
- $a_0$ which is the intercept of the oblique asymptote.

**12.** The device according to claim 11, wherein:

- the acquisition means (2a, 2b) are configured to measure a power P(t) or a developed force F(t),
- the calculation means (3) are configured to draw a Force curve F(t) as a function of the velocity v(t) of one or more muscle motor units,

governed by the following equation from Hill's muscle model:

$$(F + a)(v + b) = c$$

- these calculation means (3) are configured to determine the following metabolic parameters from this Force curve as a function of the velocity of one or more muscle motor units:

- Isometric force $F_{iso}$ corresponding to the force at zero velocity,
- Feedback resistance $R_{fb}$ corresponding to $F_{iso}/vx$ with vx equal to the velocity at zero force,
- Threshold metabolic intensity $I_T$,

With the following equations:

1. $I_T = b$
2.

$$R_{fb} = \frac{a_0}{I_T} = \frac{a_0}{b}$$

3.

$$F_{iso} = \frac{c}{I_T} - a_0 \, .$$

**13.** The device according to one of claims 11 or 12 wherein the calculation means (3) are configured to determine a figure of merit $f_m$ equal to $f_m = (R_{fb}*I_T*F_{iso}) / (R_M*B)$ .

**14.** The device according to one of claims 11 to 13, wherein the test device (1) is a treadmill or bike.

**15.** The device according to one of claims 11 to 14, wherein:

- the acquisition means (2a, 2b) are configured to measure a power P(t) or a force F(t),
- the effort test device (1) is configured to subject the subject to a variable load $Z_{load}(t)$ which is a time-modulated instantaneous load;
- the calculation means (3) are configured to determine the metabolic parameters of one or more muscle motor units, by:

- a calculation of the Laplace transforms F(p) and V(p) from the force F(t) and the velocity v(t);
- a calculation of the impedances Z (p) =F (p)/V (p) ;
- a calculation from impedances Z(p), of the following metabolic parameters:

○ Isometric force $F_{iso}$,
○ Resistance impedance of the body $Z_{IM}(p)$

the body being represented by the isometric force and the resistance impedance of the body so that:

$$F(p) = F_{iso} * Z(p) / (Z_{IM}(p) + Z(p))$$

$$V(p) = F_{iso} / (Z_{IM}(p) + Z(p))$$

• the calculation of the real part which is the metabolic parameter $R(I_M)$ from $Z_{IM}(p)$ and then the following metabolic parameters:

○ Feedback resistance $R_{fb}$;
○ Threshold metabolic intensity $I_T$;
○ Basal power B;
○ Dissipative metabolic resistance $R_M$.

16. The device according to the preceding claim, wherein the test device has real-time controlled braking to produce time-modulated braking over a frequency range of approximately $10^{-2}$ Hz to 10 Hz.

17. The device according to the preceding claim, wherein the braking is produced by a motor, mechanically coupled to a driven wheel of the test device, and which acts as an active load.

Figure 1

Figure 2

# COT

$$COI = \boxed{R_{fb}I_T} + \boxed{R}I + \frac{\boxed{B}}{I} \qquad \boxed{I_{COI_{min}}} = \sqrt{\frac{B}{R}}$$

$$\boxed{a} = a_0 + Rv \qquad \boxed{COI_{min}} = R_{fb}I_T + 2\sqrt{RB}$$

Figure 3.a

# HILL

Pente initiale: $\left[\frac{F_B}{v_T} + R_{fb} + R\right]$

Creusement: $v_T$

$V_{sc} = F_B / R_{fb}$

- $F(v) = \frac{c}{(v+b)} - a$

$a = a_0 + Rv$

$b = v_T$

$c = (F_B + a_0)\, v_T$

Figure 3.b

Figure 4

Figure 5

Figure 6

$$f_M = \frac{R_{fb} F_{iso} I_T}{R_M B}.$$

Puissance Basale B$^{-1}$

I2

Force isométrique Fiso

Intensité métabolique de seuil IT

I1

Résistance de contre-réaction Rfb

Résistance d'écoulement

EP 3 791 776 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **D. ABE et al.** Energy cost and lower leg muscle activities during erect bipedal locomotion under hyperoxia. *Journal of Physiological Anthropology, BIOMED CENTRAL L TD, LONDON, UK,* 19 Juin 2018, vol. 37, 1-11

- **H. J. RALSTON.** Energy-speed relation and optimal speed during level walking. *European Journal of Applied Physiology,* 01 Octobre 1958, vol. 17, 277-283
- **CHRISTOPHE GOUPIL et al.** *New J. Phys.,* 2019, vol. 21, 023021